# EUROPEAN PATENT APPLICATION

(11) **EP 0 557 677 A1**
(43) Date of publication of application: **01.09.1993**
(21) Application number: 92850041.2
(22) Date of filing: 24.02.1992
(51) Int. Cl.: A61F 5/44

(54) **Incontinence guard**

(71) Applicant: PROCTER & GAMBLE HYGIEN AKTIEBOLAG, S-164 93 Kista (SE)
(72) Inventor: Sten, Björnberg, S-163 53 Spanga (SE); Christer, Persson, S-578 00 Aneby (SE)
(74) Representative: Fagerlin, Heléne

(57) **Abstract**

The invention concerns an incontinence guard, characterized in that the guard is comprised of a casing (1) consisting of an outer layer (2) of soft-rigid liquid impermeable material, preferably expanded polyethylene, and an inner layer (3) of non-woven material, and optionally a liquid-absorbent insert.

## Description

The present invention relates to an incontinence guard or protector. Incontinence is the voluntary loss of bladder or bowel content, and it is a condition which affects approximately 4 % of the population in the developing countries. This means that between 10-12 million North American persons and approximately the same number of Europeans are affected by incontinence at some time in their life. Incontinence may be partial or complete, and it may be a temporary or a condition of longer-term duration. In any event, it is a symptom of a problem, not the problem itself.

The most frequent types of incontinence are: 1) stress incontinence, which relates to the passage only of a small amount of urine caused by coughing, sneezing, straining or lifting; 2) urge incontinence, generally relates to the inability of the person to reach the toilet in time to pass the urine, at which time the entire bladder contents are emptied; 3) overflow incontinence describes the spilling of a small amount of urine, even while the bladder is still full; and 4) reflex incontinence describes a loss of urine when the person is unaware of the need to urinate.

Because the general problem is very seldom discussed, statistics are still being gathered on the total scope of the problem, but generally more females than males are incontinent.

A large group of men suffering from light incontinence are those who also suffer a prostate affliction. Male incontinents normally suffer from droplet incontinence, even after having undergone prostate surgery.

Although the problem is generally dealt with, with both disposable absorbent products, as well as reusable devices, in any case it is a problem, which can result in mental suffering if the problem cannot be dealt with in a non-embarrassing method.

One-time (disposable) guards or protectors for incontinent men are known from the Swedish Patent Specification No. 426206. This known incontinence guard comprises two side walls which are mutually joined along their respective edges, such as to leave an opening along one side edge. The guard has a pointed corner and an obtuse corner. The penis of the wearer is placed in the opening so that the obtuse corner, which is located opposite the opening, is pressed against the wearer's body, wherewith the edges defining the opening are folded out towards the sides. That part of the guard which is located beneath the obtuse corner and around the pointed corner forms a funnel-shaped receptacle which is intended to receive the penis of the wearer. Since when wearing this incontinence guard the scrotum and the penis of the wearer are fully enclosed, the guard is liable to become excessively warm and too tight around the male organs, and therewith uncomfortable to wear. The incontinence guard comprises a plastic foil, an inner liquid-absorbent layer, and an outer layer made of non-woven material or like material which faces towards the male organs. This incontinence guard cannot be flushed appropriately down a toilet, and the task of ridding oneself of the soiled incontinence guard may prove embarrassing.

Another incontinence guard or protector for male urine incontinents is described and illustrated in the Swedish Patent Specification No. 416264. This incontinence guard consists of a fine-mesh seamless textile product which is impregnated with a liquid-repelling substance. Small bags containing an absorbent material are placed in the container. The incontinence guard has the form of a stocking or sock which is fitted onto the penis, which may be difficult in the case of a male whose penis is retarded, a feature which is not uncommon with older men suffering from urine incontinence. Neither can this incontinence guard be conveniently flushed down the toilet.

An insert guard or protector designed particularly for female incontinence is shown in a series of U.S. patents, such as Korpman U.S. Patent 4,685,914. Although the structure disclosed in this patent is a disposable product (intended for one-time use), it cannot be flushed down the toilet because it includes a vacuum-formed shell made of a polymer plastic with a non-woven cover of bicomponent polymer fibers secured around the periphery thereof and with a complex multiply pleated and folded urine-retaining structure inside the shell and beneath the coverstock.

The backing of this product is claimed in U.S. Patent 4,554,191, where the invention resides in a new adhesive attachment means positioned on the bottom surface of said backing for adhering the backing to the crotch portion of a nether garment. This backing is adapted to have placed therein an absorbent superstructure and is not intended for use as such. The backing is made of an ethylene containing polymer shell and does not have an inner layer as the guard according to the present invention.

Although the above descriptions relate on the one hand to male products and the other hand to female products, a great numer of disposable one-time use absorbent pads are designed for use by either male or female incontinence persons.

There are four general classifications of the goods: 1) the diaper-style, very similar to a disposable one-piece baby diaper which fits around the entire torso of the patient and fastened with elastic strands and plastic fastening tape tabs; 2) a "loin- cloth" type structure, which is generally rectangular pad which fits between the legs of the wearer and extends forwardly up to the front of the waist and reawardly up toward the back of the waist and is held in place by a pair of elastic srands fitting the ends around the waist of the wearer; 3) a 2-piece system, which includes a non-disposable (i.e. reusable) pant-like structure, which receives therein a disposable absorbent pad, generally rectangular in shape, and smaller than the loin cloth pad; and 4) the insert-type pad, which is a small "shield", similar to a large sanitary napkin, which is held in place against the crotch portion of the wearer by a specially designed reusable pant, or by the wearer's own under-garments.

In accordance with the present invention there is now provided a incontinence guard or protector comprised of a casing which comprises an outer sheet of soft-rigid liquid impermeable material, preferably expanded polyethylene, and an inner sheet of non-woven material and also optionally a liquid-absorbent insert. The guard may be used by a male or a female.

The present invention especially provides a male incontinence guard or protector with which an airy space is advantageously left around the scrotum and penis of the wearer and which can be conveniently flushed down a toilet and thereby enabling the wearer to rid himself of the guard without embarrassment.

In case of use by a female the casing preferably has a rectangular shape with rounded corners. It may have a diminished width in a region approximately midway along its length to better fit a female.

Tha casing may include a recess or hollow which may be fully or partly filled with the insert. For use by a female the hollow preferably has a depth of 0,3-4 cm, preferably 3,5-1,5 cm.

In the case of use by a male the cupped casing is intended to surround at least the penis of the wearer, and preferably both his penis and his scrotum. The casing will preferably have a round, oval, elliptical or pear-like cupped shape, and particularly a pear-like shape. The casing may be produced in several sizes, so that respective casings are able to fit men with mutually different sized male organs. The casing has a depth of 1-10 cm, preferably 3-6 cm. It may be suitable to produce the casing in three different sizes, having depths of 7, 5 and 3 cm respectively.

The protector can be used by men who suffer very slight urine incontinence. If the incontinence suffered by the wearer is manifested in only a few urinal droplets over the course of a single day, the casing can be used without the liquid-absorbing insert.

The cupped casing consists of a backing sheet of soft-rigid liquid impermeable material. By soft-rigid material is meant a material which when deformed by the application of a deforming force will return to its original shape when said force is removed, without being damaged or cracked. Preferably the material is previous to gases i.e. moisture or water vapour. The material preferably passes 300-8000, especially 1500 g water vapour/m², 24 h, 38°C. The material may be plastic materials including rubber and other polymers, especially cellular plastic materials including expanded elastomers such as natural or synthetic rubber. Thus the material may be an ethylene-containing polymer foam such as polyethylene homopolymers and ethylene-containing copolymers, preferably containing a major amount by weight of ethylene. The polymer may be cross-linked. Comonomers may be vinyl acetate, acrylic and methacrylic acids and esters or blends thereof. Preferred materials include polyethylene, polyurethane, PVC, polystyrene with closed or open cells preferably closer or partially open cells so as to breathe i.e. previous to water vapour.

The thickness and the density of the material are chosen to get the soft rigid characteristics. Most preferred is expanded polyethylene, preferably having a weight by unit of volume (volumetric weight) of 15-50 kg/m³, in particular 20-25 kg/m³, and a thickness of 0,2-5 mm, preferably 0,5-2 mm, especially 1,5 mm.

The inner layer of the casing is made of a non-woven material, preferably elastic so that it can be drawn into the backing sheet of the casing by suction when the casing is prepared. Preferably the inner layer is elastic in several directions especially in the machine direction and the cross direction. The casing is easier to produce when the non-woven has a biaxial elasticity of 2 to 50 %. The material of the inner layer in the casing is permeable to liquids.

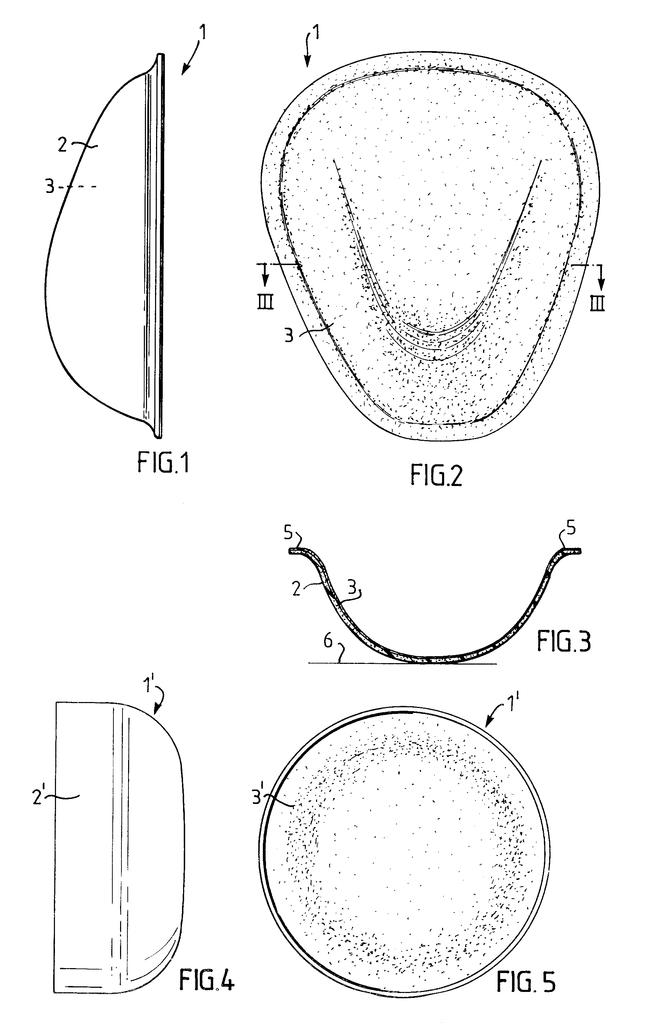

As illustrated in Figures 1-3 of the accompanying drawings, the cupped casing may have an outwardly turned edge. The edge lies against the wearer's body, therewith increasing wear comfort and reliability. This edge may have a width of 2 to 30 mm, preferably 3 to 8 mm.

The inner layer may cover the whole inner area of the cupped casing (i.e. the area exposed to the groin of a female or the penis and scrotum of a male). It may also only cover those parts thereof, that come into contact with the wearers skin. On a male product the inner layer should cover at least the areas of the backing sheet that come into contact with the mans skin. These areas are the edges of the backing sheet and that part of the cupped casing which comes into contact with the penis and scrotum. These areas are a peripherical area from the edge of the periphery and about 3 to 30 mm inwards and a central area covering the whole bottom of the cupped casing starting about 30 mm from the peripherial edge. Preferably the peripherial area is from the edge and 5-10 mm inwards and the central area begins 30-50 mm from the edge and covers the rest of the cupped casing.

When used by a female the area of the cupped casing that comes into contact with the skin is the peripherical area from the edge of the peripheri to about 3 to 30 mm inwards.

The non-woven material of the inner layer may be polypropylene, polyester, polyethylene or a bicomponent fiber containing an inner core of polypropylene and an outer cover of polyethylene. The non-woven material may be glued to the backing sheet. Preferably the materials of the backing sheet and the non-woven inner layer are chosed so as to stick to each other when heated. The rheology of the materials should preferably allow the two materials to be deepdrawn into the cupped form and stick to each other. The inner layer is preferably made of polypropylene having a melting point of about 170°C. The non-woven material preferably has a weight of 15-50 g/m² especially 20 g/m².

One advantage afforded by the cupped casing is that it can be used over several days without needing to be changed or can be disposed of after having been used only once. Thus, the casing can be washed after being used several times, allowed to dry and then used again. It can also be used together with a liquid-absorbent insert which is changed and discarded often. In this way, several days may pass before the cupped casing needs to be washed. It should be possible to use the cupped casing for more than thirty occasions of incontinence, with an exchange of the liquid-absorbent insert on each occasion. The cupped casing can normally be washed up to ten times and reused, before needing to be replaced.

The liquid-absorbing insert may be produced from any appropriate absorbent material, and preferably from a material which can be flushed down a toilet. For example, the insert may consist of toilet paper, a serviette or some other soft, absorbent paper. The insert preferably consists of two layers of non-woven material and a layer of absorbent material placed therebetween.

The absorbent material may be highly-absorbent cellular wadding, such as dry-formed cellulose, fluffed cellulose thermobonded with polyethylene, cellulose fibres or superabsorbent fibres or polymeric materials, such as Favor@ Sab 901 (salt of cross-linked polyacrylic acid), etc. Non-woven polypropylene fabric, preferably having a melting point of about 170°C, is one example of liquid-permeable material from which the outer layer of the liquid-absorbent insert can be made, although it will be understood that other liquid-permeable materials may also be used.

The liquid-absorbent insert may have any shape whatsoever, such as an oval, rectangular, round and elliptical shape. The insert may also be provided optionally with one or more slots which enable the insert to be fitted to the cupped casing more easily. These slots may have different depths and the depths of said slots may equal 5-45% of the total length of the insert in the slot direction. It will be understood that the insert may have the same shape as the outer casing and fit the wearer exactly, in which case no slots are formed.

The insert may be formed into the same form as the casing. It may contain one or more odour inhibitors.

Another advantage afforded by the novel incontinence guard is that it only takes up a small amount of space and can be used with or without an absorbent insert, or may be used with several absorbent inserts, in accordance with the needs of the wearer. The male incontinence guard is more open than the earlier known guards or urinal pads. As a result, the inventive guard is more comfortable to wear, more airy and sits more snugly than the earlier known incontinence guards, particularly when the inventive casing has a pear-like shape, this shape being better suited to the anatomical configuration of the wearer than the known incontinence guards, which fit tightly around the male organs. Although the cupped casing is made from a soft-rigid material, it is flexible when worn and will cause the wearer no embarrassment, since the casing is able to deform as the wearer moves, without breaking or rupturing. Since the absorbent insert can be replaced and is manufactured from a material which can be conveniently flushed down a lavatory, the use of the inventive incontinence guard is less embarrassing than the earlier known guards. The wearer may carry on his person spare inserts for use in replacing a soiled insert, this soiled insert being conveniently disposed of down the lavatory. This avoids the necessity of needing to rid oneself of a soiled guard in an embarrassing fashion.

The outer casing of soft-rigid material may be produced in accordance with known techniques used in the manufacture of blisters or bubble packs. Such techniques involve passing expanded plastic material and non-woven material over a frame structure having the desired shape of the outer casing, with the aid of rolls. The material is heated to softening temperature, whereafter a vacuum is applied on the inside of the mould frame so as to shape the material around said frame. The cupped shape of the casing may also be obtained by introducing the material into a matrix and shaping the casing in a heated atmosphere between the male and female parts of the matrix.

The insert is produced suitably with the aid of techniques known in the manufacture of dry-formed paper. Such techniques involve mixing 5-10% polyethylene fibres with cellulose fibres and superabsorbents in a mill, whereafter the resultant pulp is discharged onto a wire and compacted to form a web, by vacuum suction beneath the surface of the wire. The absorbent fibre web is then fed in between two webs of non-woven material and the resultant composite is introduced into an oven and there heated to a temperature of 90 ° C-150 ° C, causing the polyethylene to melt and the fibre fluff to bond together.

With the above and other objects in view, more information and a better understanding of the present invention may be achieved by reference to the following detailed description.

### Detailed description

For the purpose of illustrating the invention, there is shown in the accompanying drawings a form thereof which is at present preferred, although it is to be understood that the several instrumen- talitites of which the invention consists can be variously arranged and organized and that the invention is not limited to the precise arrangements and organizations of the instrumentalities as herein shown and described.
Figure 1 is a side view of a cupped casing having the shape of one-half of a pear;
Figure 2 is a view from above of the casing illustrated in Figure 1;
Figure 3 is a sectional view taken on the line I-I in Figure 2;
Figure 4 is a side view of a round cupped casing;
Figure 5 is a front view of the casing illustrated in Figure 4;
Figure 6 is a side view of a rectangular cupped casing with rounded corners;
Figure 7 is a front view of the casing illustrated in Figure 6;
Figure 8 shows an inventive insert from above;
Figure 9 is a sectional view taken on the line II-II in Figure 8; and
Figure 10 shows a round insert.
Figure 11 is an insert with the same form as the casing of figure 5.
Figure 12 is a casing partially covered with an inner layer.
Figure 13 shows a female casing.
Figure 14 is a female insert.

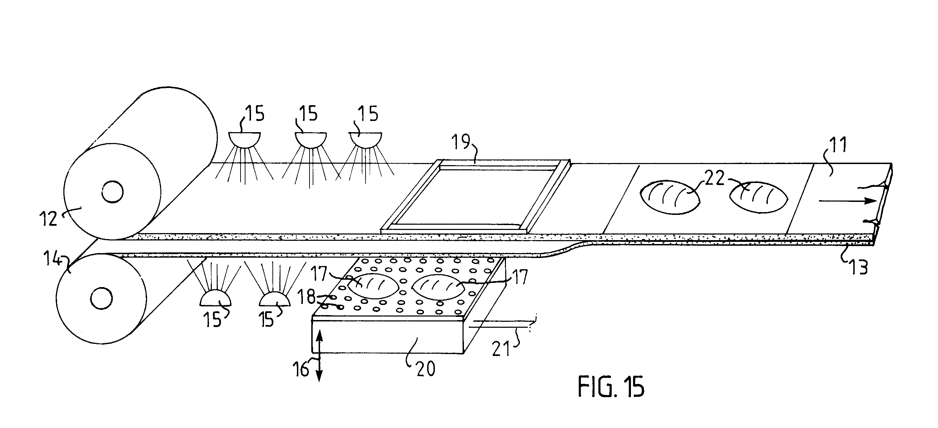

Figure 15 is a schematical representation of the production of a casing according to the example.

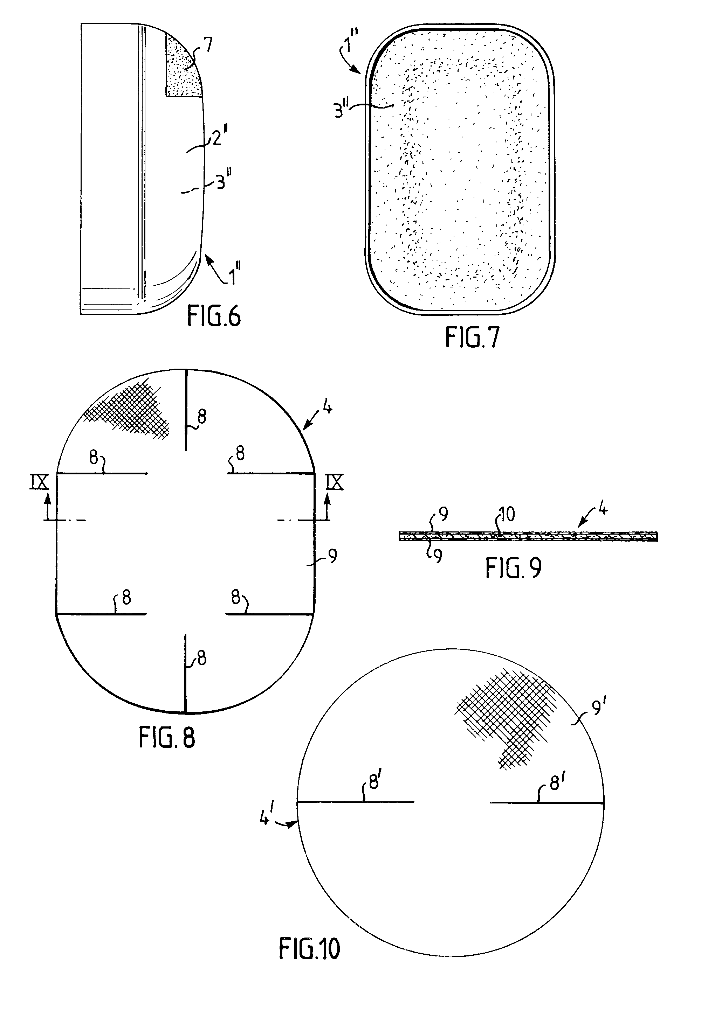

Figure 1 illustrates a cupped casing 1 which has the shape of one-half of a pear. The casing is comprised of an outer layer 2 of a soft-rigid expanded polyethylene, which is provided with an inner sheet 3 of non-woven material. The inner sheet covers the whole area of the casing that is turned inwards. Figure 2 shows the same embodiment from above, while Figure 3 is a section view taken on the line I-I in Figure 2, so that the outer backing sheet 2 and the inner sheet 3 can be clearly seen. It will also be seen that this embodiment has an outwardly turned edge 5 which is generally parallel with the tangent 6 of the highest point of the cupped casing 1. Figure 4 illustrates another embodiment in which the cupped casing 1' is completely round and has the form of a hollow hemisphere. There is no peripheral edge. Figure 4 shows the cupped casing in side view and Figure 5 is a front view of the same embodiment. Figure 6 is a side view of another embodiment of the cupped casing 1", which in this case has a rectangular shape with rounded corners. The casing of this embodiment includes an adhesive part-surface 7, for instance a double-sided adhesive tape or a self- gripping tape (Velcro@ tape) functioning to secure the casing to the clothes of the wearer. Figure 7 is a front view of the casing shown in Figure 6.

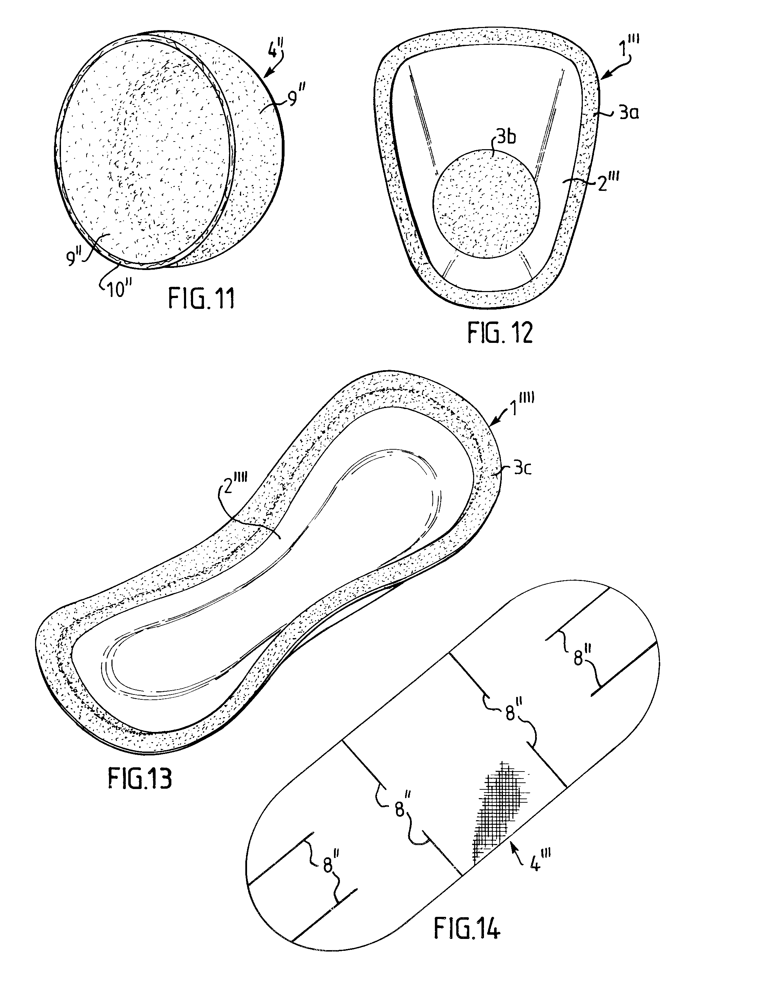

Figure 8 illustrates an exemplifying embodiment of an inventive insert 4. The Figure shows the insert from above and it will be seen from said Figure that the insert has a rectangular shape with rounded corners to fit the casing of figure 6. The insert has six slots 8, which enable the insert to be readily fitted into the casing 1". This insert also fits into the casing 1 of figure 1. Figure 9 is a sectional view taken on the line II-II in Figure 8. This Figure shows the outer layers 9 of non-woven material and the intermediate layer 10 of absorbent material. The insert illustrated in Figures 8 and 9 can be inserted into casings having the pear-like shape and oval shape illustrated respectively in Figures 1-3 and Figures 6 and 7. Figure 10 illustrates a round insert 4' having 2 slots 8'. Part of the Figure shows that the insert may be given a diamond-shaped embossed pattern.

Figure 11 shows an insert 4" with outer layers 9" of non-woven material and an intermediate layer 10" of absorbent material. This insert is formed to fit casing 1' of 4 and figure 5.

Figure 12 is a casing 1'" partially covered with an inner layer. There is a peripherical area 3a and a central area 3b respectively of inner layer. These areas of inner layers are arranged to cover those areas of the casing 1'" that will come into contact with the wearers skin.

Figure 13 is a female casing 1"" with an inner layer 3c round the edges. The inner layer 3c covers the edges that come into contact with the skin when used. The inner layer may also cover the whole inner cupped bottom of the casing. The casing has a diminished width midway along its length. The casing may also have an outwardly turned edge as is shown in figure 3 at reference number 5.

Figure 14 is an insert 4'" to be used with the female casing 1"" of figure 13. The insert has 8 slots 8".

### Example 1

A casing is prepared by moving a web 11 of expanded polyethylene with a thickness of 1 mm a weight of 20-25 kg/m³ and a softening temperature of 110-130°C from one roll 12 and another web 13 of non-woven from another roll 14 under infra heating lamps 15 (see figure 16). The non-woven consists of fibrers having an inner core of polypropylene and an outer covering layer of polyethylene with a softening temperature of 110-130°C. Web 11 is lying over web 13. The two webs are heated to 120°C so as to get soft and stick to each other.

A movable mould plate 16 with several moulds 17 and holes 18 is moved against a fixed frame 19 over the polyethylene web 11 in the direction of the arrows in figure 16. The two superimposed webs 11 and 13 are pressed against each other between the fixed frame 19 and the movable plate 16. At the same time vacuum is applied in the vacuum chamber 20 through the holes 18 and line 21 so as to form the webs round the moulds. The moulded casing which have not yet been cut or punched out is shown at 22.

## Claims

1. An incontinence guard, characterized in that the guard is comprised of a casing (1, 1', 1", 1 "', 1 "") consisting of an outer layer (2, 2', 2", 2"', 2"") of soft-rigid liquid impermeable material, and an inner layer (3, 3', 3"; 3a; 3b; 3c) of non-woven material, and optionally a liquid-absorbent insert (4, 4', 4", 4"').

2. An incontinence guard according to Claim 1, characterized in that the inner layer (3a, 3b, 3c) covers at least those parts of the guard that come into contat with the wearers skin.

3. An incontinence guard according to Claim 1 or 2, characterized in that the insert (4") is formed to the same form as the casing (1').

4. An incontinence guard according to anyone of Claims 1-3, characterized in that the casing has an outwardly turned edge (5).

5. An incontinence guard according to anyone of Claims 1-4, characterized in that the casing (1", 1 "") has a rectangular shape with rounded corners.

6. An incontinence guard according to anyone of Claims 1-5, characterized in that the casing includes a recess or hollow.

7. An incontinence guard according to Claim 6, characterized in that the hollow has a depth of 0.3-4 cm, preferably 3.5-1.5 cm.

8. An incontinence guard according to any one of the preceding Claims, characterized in that the casing (1'''') has a diminished width in a region approximately midway along its length.

9. An incontinence guard according to anyone of Claims 1-8, characterized in that the casing (1, 1', 1", 1 "') is cup-shaped and is intended to surround at least the wearer's penis or both the wearer's penis and the wearer's scrotum, and preferably has a substantially round, oval, elliptical or pear-like shape, particularly a pear-like shape.

10. An incontinence guard according to Claim 9, characterized in that the casing (1) has a depth of 1-10 cm, preferably 3-6 cm.

11. An incontinence guard according to one or more of Claims 1-10, characterized in that the insert (4) is loose and is comprised with two layers (9, 9', 9") of non-woven material and a layer (10, 10', 10") of absorbent material placed therebetween.

12. An incontinence guard according to Claim 11, characterized in that the insert (4, 4', 4") is provided with one or more slots (8, 8', 8") so as to enable the insert to be placed more readily in the casing (1).

13. An incontinence guard according to any one of Claims 11 and/or 12, characterized in that the insert (4, 4', 4", 4"') contains one or more odour inhibitors.

14. An incontinence guard according to one or more of Claims 1-13, characterized in that the casing (1") has provided on the outer surface of the outer layer (2") thereof an outwardly adhesive part-surface (7), preferably a double-sided adhesive tape.
